# EUROPEAN PATENT SPECIFICATION

(11) **EP 4 389 087 B1**
(45) Date of publication and mention of the grant of the patent: **19.08.2026**
(21) Application number: 23218587.6
(22) Date of filing: 20.12.2023
(51) Int. Cl.: A61F 9/008

(54) **LASIK CORNEAL FLAP CUTTING PATTERNS FOR BUBBLE MANAGEMENT**
LASIK-HORNHAUTLAPPENSCHNEIDMUSTER ZUR BLASENVERWALTUNG
MOTIFS DE DÉCOUPE DE RABAT CORNÉEN LASIK POUR LA GESTION DE BULLES

(30) Priority: 20.12.2022 US 202263476364 P
(43) Date of publication of application: 26.06.2024
(73) Proprietor: AMO Development, LLC, Irvine, CA 92618 (US)
(72) Inventor: VOORHEES, Andrew, Irvine, 92618 (US); FU, Hong, Irvine, 92618 (US); HILL, James, Irvine, 92618 (US); RAHAMAN, Mohammad Saidur, Irvine, 92618 (US); GAO, Wenzhi, Irvine, 92618 (US); SCHWAM, Brian, Irvine, 92618 (US); GRAY, Paul, Irvine, 92618 (US); VILLANUEVA, Cynthia, Irvine, 92618 (US); MEHTA-HURT, Deepali, Irvine, 92618 (US); NELSON, Jesse, Irvine, 92618 (US); LARON, Michal, Irvine, 92618 (US)
(74) Representative: Carpmaels & Ransford LLP

(56) References cited:
- WO-A1-2016/209312
- US-A1- 2015 190 282
- US-A1- 2016 008 173
- US-A1- 2022 175 581

## Description

### BACKGROUND OF THE INVENTION

### Field of the Invention

This invention relates to ophthalmic laser surgeries, and in particular, it relates to flap cutting in LASIK (laser-assisted in situ keratomileusis) surgery using an ultrafast resonant scanning femtosecond laser.

### Description of Related Art

Femtosecond lasers are used to cut flaps in the corneal stroma as the first step of LASIK (laser-assisted in situ keratomileusis) surgeries. A flap is typically formed by a bed cut which is parallel to the anterior corneal surface and a vertical side cut around the periphery of the bed cut expect for an uncut hinge region.

When using femtosecond lasers to cut a flap in the corneal stroma as a part of a LASIK procedure, the interaction of the laser pulses with the tissue can sometimes create excessive gas bubbles which can interfere with the continued cutting of the tissue, creating tissue bridges and rough bed cut surfaces. For example, when forming a flap bed cut in the tissue, gas bubbles may be created by the laser during previous laser raster scans, and may block laser beam in the current scan pass. This could lead to a region of uncut tissue. Gas bubbles can also shadow the laser beam for the subsequent segments of the flap cut, creating tissue bridges.

Commonly owned U.S. Pat. Appl. Pub. No. 20220175581, entitled "LASIK Flap Cutting Patterns Including Intrastromal Pocket for Bubble Management," describes "A method implemented in an ophthalmic surgical laser system that employs a resonant scanner, scan line rotator, and XY- and Z-scanners, for forming a corneal flap in a patient's eye with improved bubble management during each step of the flap creation process. A pocket cut is formed first below bed level, followed by the bed connected to the pocket cut, then by a side cut extending from the bed to the anterior corneal surface. The pocket cut includes a pocket region located below the bed level and a ramp region connecting the pocket region to the bed. The bed is formed by a hinge cut and a first ring cut at lower laser energies, followed by a bed cut and then a second ring cut, which ensures that any location in the flap bed is cut twice to minimize tissue adhesion. The side cut is formed by multiple side-cut layers at different depths which are joined together. All cuts are formed by scanning a laser scan line generated by the resonant scanner." (Abstract.) Further approaches to forming a corneal flap cut using a surgical laser are disclosed in US2015190282A1 and WO2016209312A1.

### SUMMARY

The present invention is directed to an apparatus and related computer program product for cutting a corneal flap in LASIK surgery that improves the cutting procedure. The invention is defined by the appended independent claims, and certain embodiments are defined by the appended dependent claims.

An object of the flap cutting procedure enabled by the present invention is to ensure that the entire flap bed is cut, to improve the ease of flap lift, and to reduce the likelihood of tissue tags and opaque bubble layer.

Embodiments of this invention provide flap cutting patterns including intrastromal pockets that can be implemented with a resonant scanning femtosecond laser. These flap cutting patterns will allow for gas bubbles to collect and vent posterior to and outside of the flap bed cut and side cut.

Additional features and advantages of the invention will be set forth in the descriptions that follow and in part will be apparent from the description, or may be learned by practice of the invention. The objectives and other advantages of the invention will be realized and attained by the structure particularly pointed out in the written description and claims thereof as well as the appended drawings.

To achieve the above objects, the present invention provides an ophthalmic surgical laser system as defined by appended independent claim 1

In another aspect, the present invention provides a computer program product as defined in appended independent claim 16.

It is to be understood that both the foregoing general description and the following detailed description are exemplary and explanatory and are intended to provide further explanation of the invention as claimed. It will be appreciated that where methods implemented in an ophthalmic surgical laser system of incising a cornea of a patient's eye to form a corneal flap are provided in the following detailed description, the present disclosure also provides a corresponding computer program product comprising instructions which, when executed by a controller in the ophthalmic surgical laser system, cause the ophthalmic surgical laser system to perform that method.

### BRIEF DESCRIPTION OF THE DRAWINGS

Figures 1A-1E schematically illustrate a flap procedure for forming a corneal flap according to an embodiment of the present invention.
Figure 2 schematically illustrates a corneal flap according to another embodiment of the present invention.
Figures 3A-3C schematically illustrates overlapping scan sweeps when forming a side cut of the corneal flap according to embodiments of the present invention.
Figures 4A-4B schematically illustrate a previously disclosed flap procedure for forming a corneal flap.
Figure 5 schematically illustrates raster scan sweeps formed using a fast-scan-slow-sweep scanning scheme implemented by an ultrafast raster scanning femtosecond laser.
Figures 6A and 6B schematically illustrate two exemplary ultrafast raster scanning femtosecond ophthalmic laser systems which may be used to implement embodiments of the present invention.

### DETAILED DESCRIPTION OF PREFERRED EMBODIMENTS

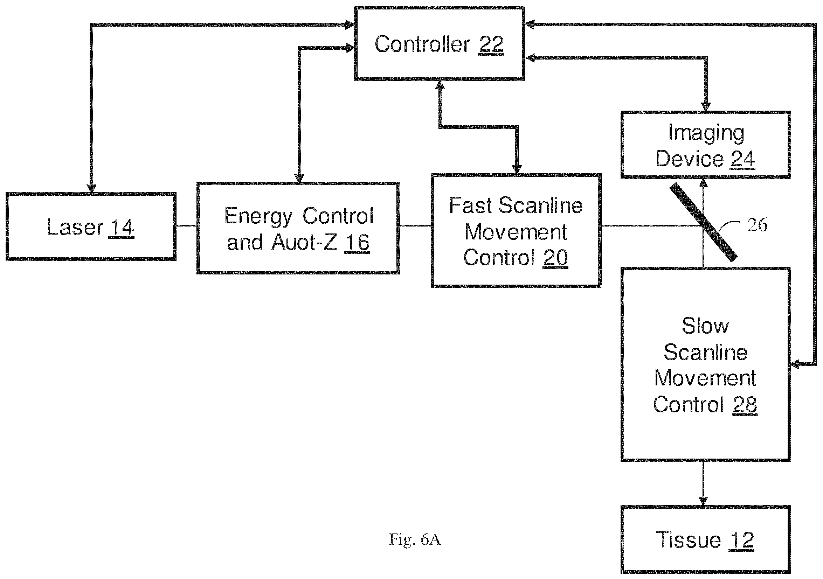

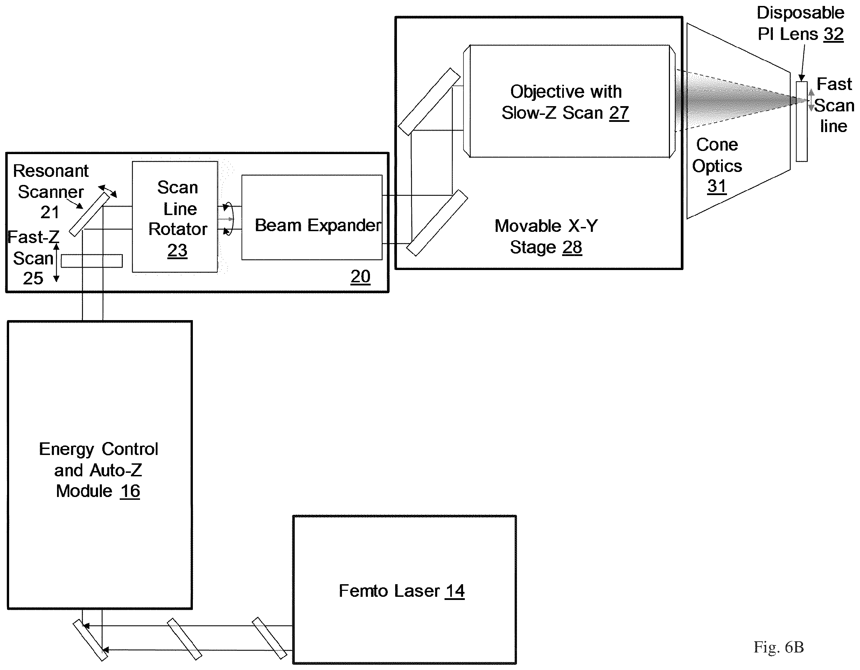

Figs. 6A and 6B illustrate ultrafast raster scanning femtosecond ophthalmic laser systems that may be used to implement the flap formation procedures according to various embodiments of the present invention.

Fig. 6A shows a system 10 for making an incision in a tissue 12 of a patient's eye, such as a cornea. The system 10 includes, but is not limited to, a laser 14 capable of generating a pulsed laser beam, an energy control module 16 for varying the pulse energy of the pulsed laser beam, a fast scan line movement control module 20 for generating the scan line of the pulsed laser beam, a controller 22, and a slow scan line movement control module 28 for moving the laser scan line and delivering it to the tissue 12. The controller 22, such as a processor operating suitable control software, is operatively coupled with the fast scan line movement control module 20, the slow scan line movement control module 28, and the energy control module 16 to direct the scan line of the pulsed laser beam along a scan pattern on or in the tissue 12. In this embodiment, the system 10 further includes a beam splitter 26 and a imaging device 24 coupled to the controller 22 for a feedback control mechanism (not shown) of the pulsed laser beam. Other feedback methods may also be used. In an embodiment, the pattern of pulses may be summarized in machine readable data of tangible storage media in the form of a treatment table. The treatment table may be adjusted according to feedback input into the controller 22 from an automated image analysis system in response to feedback data provided from a monitoring system feedback system (not shown).

Laser 14 may comprise a femtosecond laser capable of providing pulsed laser beams, which may be used in optical procedures, such as localized photodisruption (e.g., laser induced optical breakdown). Localized photodisruptions can be placed at or below the surface of the tissue or other material to produce high-precision material processing. For example, a micro-optics scanning system may be used to scan the pulsed laser beam to produce an incision in the material, create a flap of the material, create a pocket within the material, form removable structures of the material, and the like. The term "scan" or "scanning" refers to the movement of the focal point of the pulsed laser beam along a desired path or in a desired pattern.

In other embodiments, the laser 14 may comprise a laser source configured to deliver an ultraviolet laser beam comprising a plurality of ultraviolet laser pulses capable of photodecomposing one or more intraocular targets within the eye.

Although the laser system 10 may be used to photoalter a variety of materials (e.g., organic, inorganic, or a combination thereof), the laser system 10 is suitable for ophthalmic applications in some embodiments. In these cases, the focusing optics direct the pulsed laser beam toward an eye (for example, onto or into a cornea) for plasma mediated (for example, non-UV) photoablation of superficial tissue, or into the stroma of the cornea for intrastromal photodisruption of tissue. In these embodiments, the surgical laser system 10 may also include a lens to change the shape (for example, flatten or curve) of the cornea prior to scanning the pulsed laser beam toward the eye.

Fig. 6B shows another exemplary diagram of the laser system 10. Fig. 6B shows components of a laser delivery system including a moveable XY-scanner (or movable XY-stage) 28 of a miniaturized femtosecond laser system. In this embodiment, the system 10 uses a femtosecond oscillator, or a fiber oscillator-based low energy laser. This allows the laser to be made much smaller. The laser-tissue interaction is in the low-density-plasma mode. An exemplary set of laser parameters for such lasers include pulse energy in the 40-100 nJ range and pulse repetitive rates (or "rep rates") in the 2-40 MHz range, or more preferably, above 5 MHz. A fast-Z scanner 25 and a resonant scanner 21 direct the laser beam to a scan line rotator 23. When used in an ophthalmic procedure, the system 10 also includes a patient interface design that has a fixed cone nose 31 and a contact lens 32 that engages with the patient's eye. A beam splitter may be placed inside the cone 31 of the patient interface to allow the whole eye to be imaged via visualization optics. In some embodiments, the system 10 may use: optics with a 0.6 numerical aperture (NA) which would produce 1.1 µm Full Width at Half Maximum (FWHM) focus spot size; and a resonant scanner 21 that produces 0.2-1.2 mm scan line with the XY-scanner scanning the resonant scan line to a 1.0 mm field. The scan line rotator 23 (e.g., a Dove or Pechan prism, a set of mirrors, or the like, mounted on a rotating stage) rotates the resonant scan line to any direction on the XY plane. The fast-Z scanner 25 sets the incision depth. The slow scan line movement control module employs a movable XY-stage 28 carrying an objective lens with Z-scanning capability 27, referred to as slow-Z scanner because it is slower than the fast-Z scanner 25. The movable XY-stage 28 moves the objective lens to achieve scanning of the laser scan line in the X and Y directions. The objective lens changes the depth of the laser scan line in the tissue. The energy control and auto-Z module 16 may include appropriate components to control the laser pulse energy, including attenuators, etc. It may also include an auto-Z module which employs a confocal or non-confocal imaging system to provide a depth reference. The miniaturized femtosecond laser system 10 may be a desktop system so that the patient sits upright while being under treatment. This eliminates the need of certain opto-mechanical arm mechanism(s), and greatly reduces the complexity, size, and weight of the laser system. Alternatively, the miniaturized laser system may be designed as a conventional femtosecond laser system, where the patient is treated while lying down.

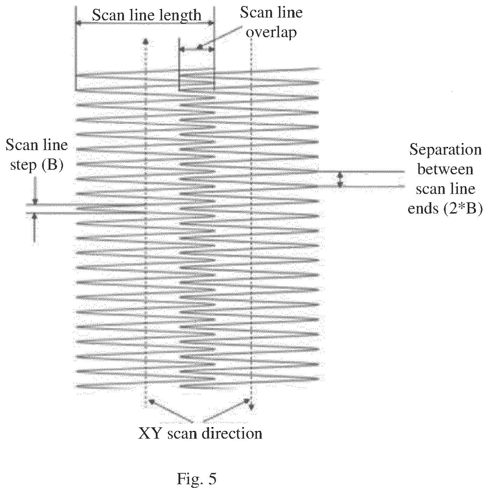

In preferred embodiments, the beam scanning can be realized with a "fast-scan-slow-sweep" scanning scheme, also referred herein as a fast-scan line scheme. The scheme consists of two scanning mechanisms: first, a high frequency fast scanner (e.g., the resonant scanner 21 of Fig. 6B) is used to scan the beam back and forth to produce a short, fast scan line; second, the fast scan line is slowly swept by much slower X, Y, and Z scan mechanisms (e.g. the moveable X-Y stage 28 and the objective lens with slow-Z scan 27, and the fast-Z scanner 25). For example, the laser system may use an 8 kHz (e.g. between 7 kHz and 9 kHz, or more generally, between 0.5 kHz and 20 kHz, or greater than 1 kHz) resonant scanner 21 to produce a fast scan line of about 1 mm (e.g., between 0.9 mm and 1.1 mm, or more generally, between 0.2 mm and 1.2 mm) and a scan speed of about 25 m/sec, and X, Y, and Z scan mechanisms with the scan speed (sweeping speed) smaller than about 0.1 m/sec. The fast scan line may be perpendicular to the optical beam propagation direction, i.e., it is always parallel to the XY plane. The trajectory of the slow sweep can be any three dimensional curve drawn by the X, Y, and Z scanning devices (e.g., XY-scanner 28 and fast-Z scanner 25). Fig. 5 schematically illustrates two parallel, overlapping scan sweeps formed by the fast-scan-slow-sweep scanning scheme.

An advantage of the "fast-scan-slow-sweep" scanning scheme is that it only uses small field optics (e.g., a field diameter of 1.5 mm) which can achieve high focus quality at relatively low cost. The large surgical field (e.g., a field diameter of 10 mm or greater) is achieved with the XY-scanner, which may be unlimited.

As described in more detail below, the flap procedures in various embodiments of the present invention utilize the fast-scan-slow-sweep scanning scheme to form various cuts of the flap. The controller of the laser system controls the various components of the system to form various cuts described below.

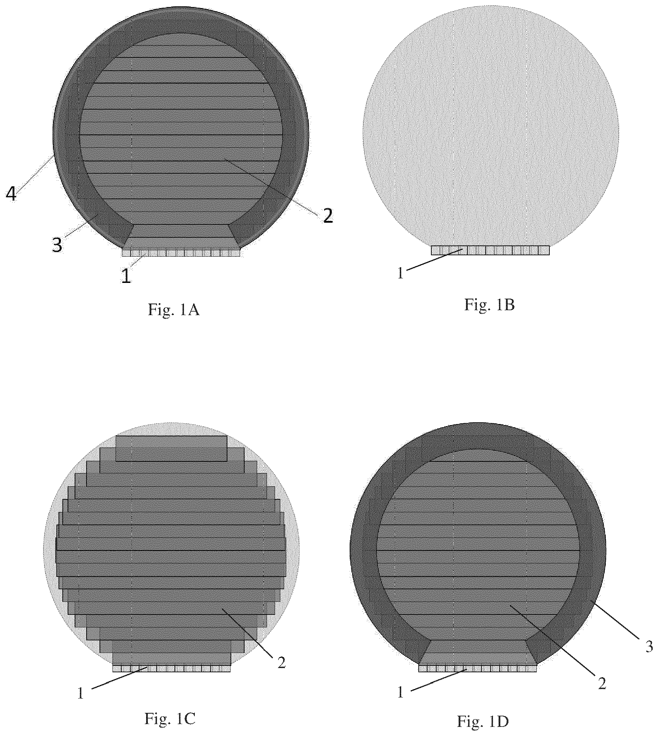

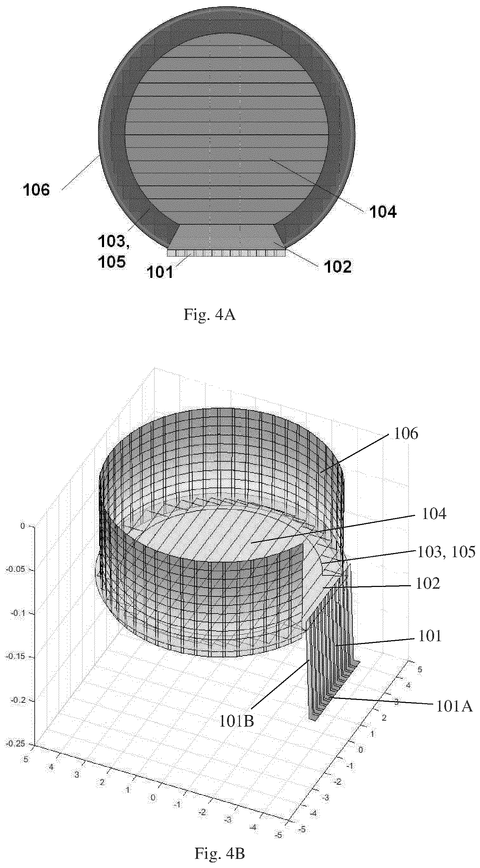

A previously disclosed technique for managing gas bubble formation during bed cut for a corneal flap, described in the above-mentioned U.S. Pat. Appl. Pub. No. 20220175581 ("the '581 publication"), is shown in Figs. 4A (top view) and 4B (perspective view), adapted from Figs. 1A-1B of the '581 publication. The flap procedure includes the following components, in the cutting order: a pocket cut 101 with a ramp, a low energy hinge cut 102, a first (low energy) ring cut 103, a bed cut 104, a second (normal energy) ring cut 105, and a side cut 106. The hinge cut 102, ring cuts 103 and 105, and bed cut 104 are located on the same plane, parallel to the anterior corneal surface, to form the bed of the flap. The overall shape of the bed is a circular area except for an uncut arc segment serving as the hinge portion of the flap (the straight line of the arc segment may be referred to as the hinge line). The side cut 106, which extends from the bed upwards to the anterior corneal surface to form the side of the flap, is located around the entire periphery of the bed except for an uncut arc at the hinge. The pocket cut 101 is located at the hinge line of the bed and extends below the bed level.

The pocket cut 101 includes a pocket region 101A and a ramp region 101B. The ramp region 101B is aligned along the hinge line of the flap in the top view, and extends from a depth slightly above the bed level (e.g., 2 to 20 µm above the bed level) to a depth about 70 to 150 µm below the bed level. The pocket region 101A extends substantially horizontally from the lower end of the ramp region 101B for about 150 to 400 µm. The pocket cut 101 preferably extends along the entire length of the hinge line, or along a part of the length of the hinge line. The ramp region 101B may be vertical, or alternatively be inclined (e.g., within 0-170 degrees from the vertical direction). Overall, the pocket cut 101 has a rectangular shape when viewed from the top, and also has a rectangular shape in a side view when viewed along a direction perpendicular to the hinge line.

The pocket cut 101 is made first, with the ramp region connecting to the bed level of the bed cut to be formed, along the hinge line. The pocket cut may be made by placing the laser scan line parallel to the hinge line and scanning the scan line along the intended surface of the pocket cut using the XY scanner and the Z scanner. The scanning direction is radially inwardly for the pocket region 101A and in a deep-to-shallow direction for the ramp region 101B. Multiple passes may be executed side-by-side (preferably with edge overlaps) to form the entire pocket.

Then, a cutting pass is made at the bed level, located across the hinge portion along the hinge line, forming the hinge cut 102 which connects to the pocket cut. A low energy ring cut 103 is made at the bed level along the periphery of the bed circle except for the hinge portion, to create the peripheral edge of the flap bed. The ring cut 103 is made by placing the laser scan line in a radial direction at the required distance from the center of the bed, so that its outer end is located at the circumference of the bed circle, and scanning the scan line using the XY scanner along the circumferential direction while using the scan line rotator to rotate the scan line direction to keep it in the radial direction. The order of the hinge cut 102 and ring cut 103 may be reversed. Both cuts are made at laser pulse energy levels that are lower than the other cutting steps, e.g., at 90% (or more generally, from 85% to 95%) of the pulse energy of the other cutting steps. These lower energy cuts generate less bubbles and less distortion of the tissue. In preferred embodiments, the pulse energy for the pocket cut, the bed cut, the second ring cut, and the side cut is 40 to 90 nJ.

Then, the bed cut 104 is made by creating overlapping parallel raster scan passes of the laser scan line, covering substantially the entire bed circle (at least all the areas not covered by the ring cut 103) except for the arc segment of the hinge. Preferably, adjacent parallel raster scan passes overlap with each other in the width direction by at least 50% of their widths, so that in effect, each point is covered by at least two passes. Note that one of the scan passes preferably overlaps with the low energy hinge cut 102. The second ring cut 105 is then made in the same area of the first ring cut 103 to ensure tissue separation at the edges of the bed. The second ring cut 105 is made in the same way as the first ring cut 103 but at the normal pulse energy.

The hinge cut 102, first ring cut 103, bed cut 104, and second ring cut 105 overlap each other so that any given point within the bed is covered by at least two passes, which minimize residual uncut tissue bridges. Moreover, the cutting sequence of the hinge cut 102, first ring cut 103, bed cut 104, and second ring cut 105 ensures that a venting channel is always present that connects the current cutting point through earlier-formed cuts to the pocket, so that the gas formed at the current cutting point always has somewhere to escape to, thereby voiding opaque bubble layer formation.

Lastly, a side cut 106 is made. The side cut may be formed by placing the laser scan line along the circle of the side cut in a tangential direction, and scanning the scan line in the vertical or near vertical direction using the Z scanner (and optionally the XY scanner) of the ophthalmic laser system. After each vertical scan, the scan line is moved by the XY scanner to the next position along the circle and rotated by the scan line rotator to be again tangent to the circle, and the vertical scanning is repeated. In alternative embodiments, the side cut 106 is formed in using a multilayer technique, described in more detail later.

The cutting order described above, which cuts the ring cut and hinge cut twice, the first time at lower energy, has the advantage of avoiding tissue bridges. The low energy hinge cuts 102 and low energy ring cut 103 generate less bubbles and less distortion of tissue.

A corneal flap procedure for forming a corneal flap according to an embodiment of the present invention is described now with reference to Figs. 1A-1E. As shown in Figs. 1A (top view) and 1E (perspective view), the flap procedure includes the following components, in the cutting order: a pocket cut 1 with a ramp, a bed cut 2, a ring cut 3, and a side cut 4. The bed cut 2 and ring cut 3 are located on the same plane, parallel to the anterior corneal surface, to form the bed of the flap. The overall shape of the bed is a circular area except for an uncut arc segment serving as the hinge portion of the flap (the straight line of the arc segment may be referred to as the hinge line). The pocket cut 1 is located at the hinge line of the bed and extends below the bed level. The side cut 4, which extends from the bed upwards to the anterior corneal surface to form the side of the flap, is located around the entire periphery of the bed except for an uncut arc at the hinge. The side cut 4 may be vertical (perpendicular to the bed) or non-vertical. Preferably, the side cut 4 forms an angle of 30° to 150° with respect to the bed.

The pocket cut 1 includes a pocket region 1A and a ramp region 1B. In this example, the ramp region 1B extends substantially vertically parallel to the hinge line of the flap, but shifted slightly from the hinge line toward the bed center in the top view. The ramp region 1B extends from a depth at the bed level to a depth about 70 to 150 µm, preferably about 100 µm, below the bed level. The pocket region 1A extends substantially horizontally from the lower end of the ramp region 1B for about 150 to 400 µm, preferably about 200 µm. The pocket cut 1 preferably extends along the entire length of the hinge line, or along a part of the length of the hinge line. Although the ramp region 1B is substantially vertical in the illustrated embodiment, it may alternatively be non-vertical (e.g., within 0-170 degrees from the vertical direction). Overall, the pocket cut 1 has a rectangular shape when viewed from the top, and also has a rectangular shape in a side view when viewed along a direction perpendicular to the hinge line.

To form the flap, the pocket cut 1 is made first (Fig. 1B), with the ramp region reaching the bed level of the bed cut to be formed near the hinge line (the circle in Fig. 1B depicts the bed to be formed). The pocket cut 1 may be made by placing the laser scan line parallel to the hinge line at the pocket depth and scanning the scan line along the intended surface of the pocket cut using the XY scanner and the Z scanner of the ophthalmic laser system. The scanning direction is first radially inwardly for the pocket region 1A and then in a deep-to-shallow direction for the ramp region 1B. Multiple passes may be executed side-by-side (preferably with edge overlaps) to form the entire pocket spanning the length of the hinge. The pocket cut 1 is optional.

Next, the bed cut 2 is made (Fig. 1C) by creating overlapping parallel raster scan passes of the laser scan line, covering substantially the entire bed circle except for the arc segment of the hinge. Preferably, adjacent parallel raster scan passes (represented by the rectangular shapes in Fig. 1C) overlap with each other in the width direction by 20 to 80% of their widths. In some embodiments, the overlap is at least 50%, so that in effect, each point is covered by at least two passes. The raster scan passes are preferably parallel to the hinge line, with the first pass located along the hinge line and having the same length as the pocket, and the other passes sequentially shifted away from the hinge line.

The ring cut 3 is made next along the periphery of the bed circle except for the hinge portion (Fig. 1D), to create the peripheral edge of the flap bed. The two ends of the ring cut 3 are preferably located at the edge of the pocket cut 1. The ring cut 3 covers the jagged edges resulting from the multiple passes of the bed cut, and ensures tissue separation at the circular edge of the flap bed. The ring cut 3 is made by placing the laser scan line in a radial direction at the required distance from the center of the bed, so that its outer end is located at the circumference of the bed circle, and scanning the scan line using the XY scanner along the circumferential direction while using the scan line rotator to rotate the scan line direction to keep it in the radial direction.

Lastly, the side cut 4 is made (Fig. 1A). In some embodiments, the side cut may be formed by placing the laser scan line along the circle of the side cut in a tangential direction, and scanning the scan line in the vertical (or non-vertical) direction using the Z scanner (or both the Z scanner and the XY scanner). After each scan, the scan line is moved by the XY and Z scanners to the next position along the circle and rotated by the scan line rotator to be again tangent to the circle, and the scanning is repeated. In alternative embodiment, the side cut 4 is formed using a multilayer technique, described in more detail later.

In preferred embodiments, the pulse energy for forming the pocket, bed, ring and side cuts is less than 100 nJ, or more preferably, from 40 to 90 nJ, and the pulse energy of the bed cut and the side cut may be set independently. The scan line length is between 400 and 1100 µm. In preferred embodiments, the speed of the XY and Z movements of the scan line is such that the distance traversed during a single period of the high frequency scanner is less than 6 µm (see Fig. 5, where the distance traversed during a single period of the high frequency scanner is 2*B).

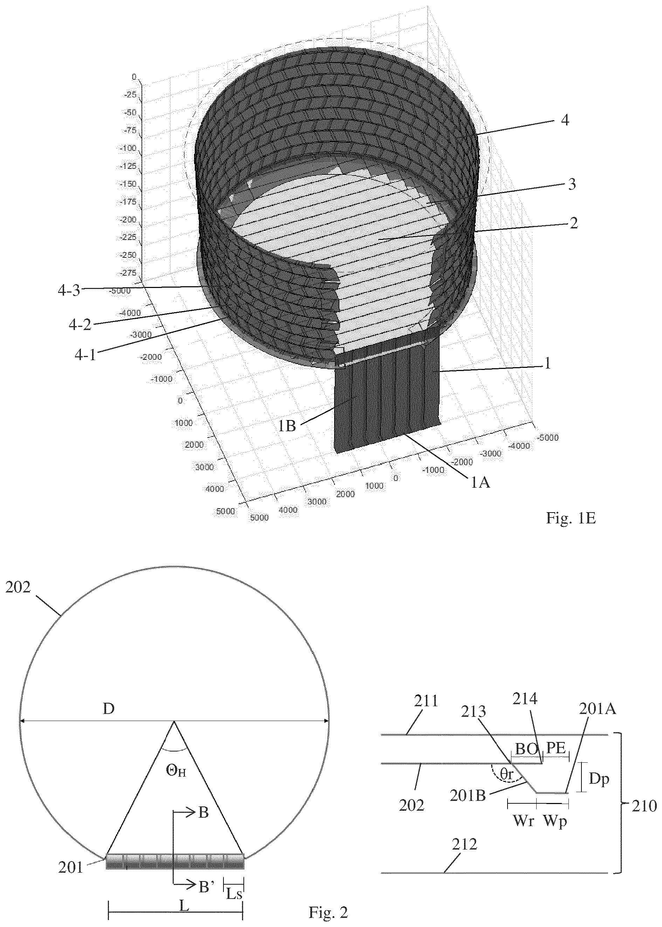

Note that although in Fig. 1E the ramp region 1B is shown as extending slightly above the bed level and intersecting the bed along a line parallel to the hinge line but closer to the bed center, in alternative embodiments, the ramp region may terminate at the bed level and/or intersect the bed at the hinge line.

Although in Figs. 1A-1E the shape of the bed is a circle except for the hinge portion, in alternative embodiments, the bed may be an elliptical shape except for the hinge portion.

Compared to the cutting procedure described in the '581 publication (Figs. 4A-4B), in the above-described embodiment of the present invention (Figs. 1A-1E), a low energy hinge cut and a first low energy ring cut are not performed; the bed is formed by only the bed cut 2 and the single ring cut 3, performed at the same pulse energy. In the above embodiment, the execution sequence for the various cuts ensures that the first pass of the bed intersects the entire pocket ramp, thus creating a wider and easier path for gas to flow away from the subsequent bed cut scans. Further the overlap of the subsequent parallel bed scans continues to allow the gas to have an easy path to the pocket. This is an improvement over the prior pattern described in Figs. 4A-4B where the initial portion of the bed is the ring cut which only intersects a small portion of the pocket ramp and all gas must move back to the pocket through a smaller entry. The above embodiment significantly reduces the likelihood that opaque bubble layer will interfere with the formation of the bed cut. In a clinical validation study, the cutting procedure according to the embodiment of the present invention provided significantly improved ease of flap lift as compared to the cutting procedure described in the '581 publication. It also reduces the number of cutting steps and reduces the procedure time..

Fig. 2 (top view and depth profile) schematically illustrates a flap procedure that forms a corneal flap with a pocket cut according to another embodiment of the present invention. As shown in Fig. 2, the flap is formed of a bed cut 202, which is a circular or elliptical area except for an uncut arc segment serving as a hinge portion, a pocket cut 201 connected to the bed cut near the hinge line, including a ramp region 201B and a pocket region 201A, and a side cut (also designated by the reference symbol 202) located around the periphery of the bed cut except for the uncut hinge portion. The pocket cut 201 (the ramp region 201B and the pocket region 201A collectively) has a rectangular shape in the top view, with a length L approximately equal to the length of the hinge portion.

Fig. 2 also illustrates a depth profile 210 through the center of the hinge as indicated by arrows B-B', showing the anterior corneal surface 211, posterior corneal surface 212, the ramp region 201B, the pocket region 201A, and a part of the bed cut 202. The depth profile is the same throughout the entire length L of the pocket cut 201. As seen in the depth profile, the pocket region 201A is located below (deeper than) the bed cut 202, the upper-inner end of the non-vertical ramp region 201B is connected to the bed cut 201 at a location 213 that is offset from the hinge line 214 toward the bed center, and the lower-outer end of the ramp region 201B is connected to the pocket region 201A. This way, the ramp region 201B connects the pocket region 201A to the bed cut 202. In the illustrated embodiments, the depth profile of the ramp 202 is linear, but it may alternatively have a curved shape. For example, it may have rounded connections with the pocket region 201A. As shown in Fig. 2, the portion of the bed cut 202 that extends beyond the intersection location 213 may be referred to as "bed overcut" (BO), and the portion of the pocket cut 201 that extends beyond the hinge line 214 (in the top view) may be referred to as "pocket extension" (PE).

The various cuts of the embodiment of Fig. 2 are formed in similar manners as those of the embodiment of Fig. 1A-1D.

In one particular example (see Fig. 2), the flap diameter D is 9.0 mm, the pocket width Wp is 200 µm, the pocket depth Dp (measured from the bed level) is 100 µm, the ramp angle θr relative to the horizontal plane is 150°, the ramp width Wr is 170 µm, the horizontal bed overcut BO is 250 µm, and the pocket extension PE over the hinge line is 120 µm. The hinge length is 4.2 mm, and the hinge angle Θ_{H} (the angle spanned by the hinge line with respect to the bed center) is approximately 55°. The scan line length Ls for forming the pocket cut 201 is about 600 µm, and eight parallel scans are used in this example to form the entire length of the pocket cut 201. Other appropriate parameters may be used. More generally, the flap diameter D may be between 5 and 10 mm, the pocket width Wp may be between 150 and 400 µm, and the hinge angle Θ_{H} may be between 45 and 90 degrees.

Pocket cuts of various other shaped pocket cut may be formed, some of which are described in the above-mentioned '581 publication.

In the flap cutting procedures according to the above embodiments, the pockets allow the gas bubbles to move into the pockets and out of the laser path of the subsequent cut. It is not essential for the bubbles to move all the way back to the pocket, however, so long as they move backward to earlier cut areas. Thus, for example, in a bed cut, ring cut, side cut cutting order, when cutting the side cut, it is not essential for the gas bubbles to move into the pocket; rather, it is sufficient if they move back to the ring or bed cut.

Referring back to Fig. 1E, in a preferred embodiment, the side cut 4 is formed using a multilayer technique to form a layered side cut. More specifically, the side cut 4 is formed one horizontal layer at a time, where each layer 4-1, 4-2, 4-3, etc. is a part of the overall side cut but extends only in a depth range that is a sub-range of the entire depth range of the side cut. All but the top layer are completely located within the cornea and do not reach the anterior corneal surface. The layers are aligned with each other along the entire depth range and connect with each other to form the side cut. The adjacent layers preferably overlap each other in the depth direction (i.e., their sub-ranges overlap each other). In one example, the layer thickness, i.e. the size of the depth sub-range, is about 25 µm, and the overlap between two adjacent layers is about 10 µm. More generally, the layer thickness may be from 10 to 40 µm, and the layer overlap may be from 2 to 15 µm. The bottom-most layer may extend below the bed level and the top-most layer may extend above the anterior corneal surface, to ensure proper separation of the flap. The number of side cut layers is determined by the total depth of the side cut, the thickness of each layer and the amount of overlap between adjacent layers. The example illustrated in Fig. 1E has ten layers. The different layers may also have different thicknesses.

The side cut layers are formed in a sequence from posterior to anterior (i.e. deeper to shallower relative to the anterior corneal surface), preferably changing layers at the corner of the hinge to start the subsequent side cut layer. The top of the last side cut layer that is located completely inside the cornea is preferably within less than 20 µm from the top surface of epithelium in order to manage the bubbles generated within the ring cut, bed cut and side cut.

More specifically, each side cut layer 4-1, etc. is created by placing the laser scan line tangent to the side-cut path (along the circumference), oscillating it sinusoidally in the Z direction, simultaneously moving it around the side-cut path in the circumferential XY direction, and simultaneously rotating the scan line to keep it tangent to the side-cut path. In one particular example, the Z oscillation frequency is 120 Hz, the scan line length is 600 µm, the scan line overlap is 25%, the glass overcut at the anterior corneal surface is 90 µm, the side cut angle is 120°, and the flap diameter was 8.0 mm.

Preferably, during the anterior-to-posterior half of each sinusoidal period, the laser beam is blanked using a fast blanking technique in order to manage the generated bubbles in the corneal tissue. For example, fast blanking may be achieved by temporarily reducing the laser pulse energy to below the photodisruption threshold of the corneal tissue and increasing pulse duration so that no tissue cutting occurs, which in turn may be achieved by temporarily increasing the laser pulse repetition rate while keeping the pump current of the laser constant. Thus, each posterior-to-anterior half period form a scan sweep.

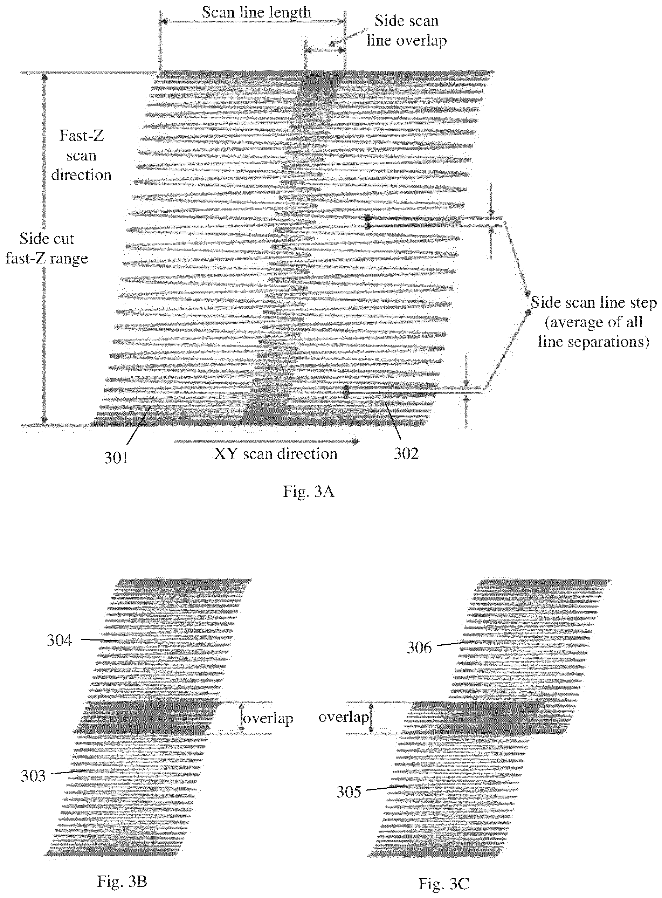

Figs. 3A schematically illustrates two adjacent posterior-to-anterior scan sweeps 301, 302 that form a part of a side cut layer, the two sweeps overlapping each other in the XY scan (horizontal) direction at their edges. Fig. 3B schematically illustrates an example of two scan sweeps 303, 304 in two adjacent side cut layers that overlap each other in the Z (depth) direction. Fig. 3C schematically illustrates an example of two scan sweeps 305, 306 in two adjacent side cut layers that overlap each other in the Z (depth) direction; the two scan sweeps in this example are also shifted relative to each other in the XY scan direction.

It will be apparent to those skilled in the art that various modification and variations can be made in the corneal flap procedure and related apparatus of the present invention without departing from the scope of the invention. Thus, it is intended that the present invention cover modifications and variations that come within the scope of the appended claims.

## Claims

1. An ophthalmic surgical laser system, comprising:
a laser delivery system configured to deliver a pulsed laser beam to a cornea of a patient's eye;
a high frequency scanner (21) configured to scan the pulsed laser beam back and forth at a predefined frequency to form a laser scan line;
a scan line rotator (23) configured to rotate an orientation of the laser scan line;
an XY-scanner (28) and a Z-scanner (27) configured to move the laser scan line in lateral and depth directions; and
a controller (22) operatively coupled to and programmed to control the scan line rotator, the XY-scanner and the Z-scanner to scan the laser scan line in the cornea to form a corneal flap, including to form a bed (202) of the flap and to form a side cut (4) of the flap, and to form a pocket cut (1) prior to forming the bed cut,
wherein the bed is located in a horizontal plane at a first depth from an anterior corneal surface, the bed defining a hinge line (214), wherein the side cut extends from the bed upwards to the anterior corneal surface to form a side of the flap, the side cut surrounding an entire periphery of the bed except the hinge line,
wherein the bed of the flap consists of a bed cut (2) formed by scanning the laser scan line in successive overlapping parallel raster scan passes, starting along the hinge line, and a single ring cut (3) along a periphery of the bed except for an area of the hinge cut formed by scanning the laser scan line along a circumference of the bed, wherein the ring cut covers all areas of the bed not covered by the bed cut, and
wherein the pocket cut extends from a second depth posterior to the bed to the depth of the bed, wherein the bed cut is connected to the pocket cut;
wherein the pocket cut includes a ramp region (1B, 201B) and a pocket region (1A, 201A) connected to each other, wherein the pocket region is located at the second depth, and wherein the ramp region extends between the first depth and the second depth and is connected to both the bed and the pocket region.

2. The system of claim 1, wherein all cuts that form the corneal flap are performed at a same laser pulse energy

3. The system of claim 1, wherein the side cut includes a plurality of side cut layers (4-1,4-2,4-3) formed in a sequence, each side cut layer extending within a depth range relative to the anterior corneal surface, wherein all except one of the plurality of side cut layers are located entirely within the cornea without reaching the anterior corneal surface, and wherein the plurality of side cut layers are aligned with each other and connect with each other to form the side cut.

4. The system of claim 3, wherein each of the plurality of side cut layers is formed by placing the laser scan line tangent to a circumference of the side cut, moving the laser scan line in a vertical direction, simultaneously moving the laser scan line around the circumference, and simultaneously rotating the scan line to keep it tangent to the circumference.

5. The system of claim 3, wherein adjacent side cut layers overlap each other in a depth direction.

6. The system of claim 1, wherein a laser pulse energy of the bed cut and a laser pulse energy of the side cut can be set independently.

7. The system of claim 1, wherein each successive one of the raster scan passes overlaps a previous scan pass by 20 to 80% of a length of the scan lines.

8. The system of claim 1, wherein the side cut forms a non-90 degree angle relative to the bed cut.

9. The system of claim 8, wherein the side cut forms an angle of from 30° to 150° relative to the bed cut.

10. The system of claim 1, further comprising a pulsed laser to generate the pulsed laser beam, optionally wherein a repetition rate of the pulsed laser is greater than 5 MHz and/or wherein a pulse energy of the pulsed laser is less than 100 nJ.

11. The system of claim 1, wherein a frequency of the high frequency scanner is greater than 1 kHz.

12. The system of claim 1, wherein a length of the scan line in the bed cut, the ring cut and the side cut is between 400 and 1100 µm.

13. The system of claim 1, wherein the controller is configured to control the XY-scanner and the Z-scanner to scan the scan line such that a distance traversed during a single period of the high frequency scanner is less than 6 µm.

14. The system of claim 1, wherein the flap has an elliptical shape.

15. The system of claim 1, further comprising, during transitions between successive scan passes of the bed and side cut, temporarily blanking pulses of the pulsed laser beam by temporarily increasing a pulse repetition rate of the pulsed laser beam which temporarily reduces the pulse energy of the pulsed laser beam to below a photodisruption threshold of a corneal tissue and increases the pulse duration.

16. A computer program product comprising instructions which, when executed by a controller of an ophthalmic surgical laser system, cause the ophthalmic surgical laser system to incise a cornea of a patient's eye to form a corneal flap, by:
controlling a laser delivery system of the ophthalmic surgical laser system to deliver a pulsed laser beam to the cornea;
controlling a high frequency scanner of the ophthalmic surgical laser system to scan the pulsed laser beam back and forth to form a laser scan line; and
controlling a scan line rotator, an XY-scanner and a Z-scanner of the ophthalmic surgical laser system to move the laser scan line in the cornea to form the corneal flap, including forming a bed of the flap and forming a side cut of the flap and prior to forming the bed cut, forming a pocket cut,
wherein the bed is located in a horizontal plane at a first depth from an anterior corneal surface, the bed defining a hinge line, wherein the side cut extends from the bed upwards to the anterior corneal surface to form a side of the flap, the side cut surrounding an entire periphery of the bed except the hinge line,
wherein forming the bed of the flap consists of:
forming a bed cut by scanning the laser scan line in successive overlapping parallel raster scan passes, starting along the hinge line, and
forming a single ring cut along a periphery of the bed except for an area of the hinge cut by scanning the laser scan line along a circumference of the bed, wherein the ring cut covers all areas of the bed not covered by the bed cut,
and the pocket cut extends from a second depth posterior to the bed to the depth of the bed, wherein the bed cut is connected to the pocket cut;
wherein the pocket cut includes a ramp region and a pocket region connected to each other, wherein the pocket region is located at the second depth, and wherein the ramp region extends between the first depth and the second depth and is connected to both the bed and the pocket region.

## Patentansprüche

1. Ophthalmologisches Chirurgielasersystem, umfassend:
ein Laserabgabesystem zum Abgeben eines gepulsten Laserstrahls an eine Hornhaut eines Patientenauges;
einen Hochfrequenzscanner (21), der konfiguriert ist, um den gepulsten Laserstrahl mit einer vordefinierten Frequenz hin und her abzutasten, um eine Laserabtastlinie zu bilden;
einen Abtastlinienrotator (23), der konfiguriert ist, um eine Ausrichtung der Laserabtastlinie zu drehen;
einen XY-Scanner (28) und einen Z-Scanner (27), die konfiguriert sind, um die Laserabtastlinie in lateraler Richtung und in Tiefenrichtung zu bewegen; und
eine Steuerung (22), die mit dem Abtastlinienrotator, dem XY-Scanner und dem Z-Scanner wirkverbunden und programmiert ist, um den Abtastlinienrotator, den XY-Scanner und den Z-Scanner zu steuern, um die Laserabtastlinie in der Hornhaut zu scannen, um eine Hornhautklappe zu bilden, einschließlich des Bildens eines Bettes (202) der Klappe und des Bildens eines Seitenschnitts (4) der Klappe, und des Bildens eines Taschenschnitts (1) vor dem Bilden des Bettschnitts,
wobei das Bett in einer horizontalen Ebene in einer ersten Tiefe von einer anterioren Hornhautoberfläche angeordnet ist, wobei das Bett eine Scharnierlinie (214) definiert, wobei sich der Seitenschnitt von dem Bett nach oben zu der anterioren Hornhautoberfläche erstreckt, um eine Seite der Klappe zu bilden, wobei der Seitenschnitt eine gesamte Peripherie des Bettes mit Ausnahme der Scharnierlinie umgibt,
wobei das Bett der Klappe aus einem Bettschnitt (2), der durch Abtasten der Laserabtastlinie in aufeinanderfolgenden überlappenden parallelen Rasterabtastdurchgängen beginnend entlang der Scharnierlinie gebildet wird, und einem einzigen Ringschnitt (3) entlang einer Peripherie des Bettes mit Ausnahme eines Bereichs des Scharnierschnitts besteht, der durch Abtasten der Laserabtastlinie entlang eines Umfangs des Bettes gebildet wird, wobei der Ringschnitt alle Bereiche des Bettes abdeckt, die nicht durch den Bettschnitt abgedeckt sind, und
wobei sich der Taschenschnitt von einer zweiten Tiefe posterior zu dem Bett bis zu der Tiefe des Bettes erstreckt, wobei der Bettschnitt mit dem Taschenschnitt verbunden ist;
wobei der Taschenschnitt einen Rampenbereich (1B, 201B) und einen Taschenbereich (1A, 201A) einschließt, die miteinander verbunden sind, wobei der Taschenbereich in der zweiten Tiefe angeordnet ist, und wobei sich der Rampenbereich zwischen der ersten Tiefe und der zweiten Tiefe erstreckt und sowohl mit dem Bett als auch mit dem Taschenbereich verbunden ist.

2. System nach Anspruch 1, wobei alle Schnitte, die die Hornhautklappe bilden, mit einer gleichen Laserpulsenergie durchgeführt werden.

3. System nach Anspruch 1, wobei der Seitenschnitt eine Vielzahl von Seitenschnittschichten (4-1,4-2,4-3) einschließt, die in einer Abfolge gebildet sind, wobei sich jede Seitenschnittschicht innerhalb eines Tiefenbereichs relativ zu der anterioren Hornhautoberfläche erstreckt, wobei sich alle bis auf eine der Vielzahl von Seitenschnittschichten vollständig innerhalb der Hornhaut befinden, ohne die anteriore Hornhautoberfläche zu erreichen, und wobei die Vielzahl von Seitenschnittschichten aneinander ausgerichtet sind und sich miteinander verbinden, um den Seitenschnitt zu bilden.

4. System nach Anspruch 3, wobei jede der Vielzahl von Seitenschnittschichten durch Platzieren der Laserabtastlinie tangential zu einem Umfang des Seitenschnitts, Bewegen der Laserabtastlinie in einer vertikalen Richtung, gleichzeitiges Bewegen der Laserabtastlinie um den Umfang und gleichzeitiges Drehen der Abtastlinie, um sie tangential zum Umfang zu halten, gebildet wird.

5. System nach Anspruch 3, wobei benachbarte Seitenschnittschichten einander in einer Tiefenrichtung überlappen.

6. System nach Anspruch 1, wobei eine Laserpulsenergie des Bettschnitts und eine Laserpulsenergie des Seitenschnitts unabhängig voneinander eingestellt werden können.

7. System nach Anspruch 1, wobei jeder aufeinanderfolgende der Rasterabtastdurchgänge einen vorherigen Abtastdurchgang um 20 bis 80 % einer Länge der Abtastlinien überlappt.

8. System nach Anspruch 1, wobei der Seitenschnitt einen Winkel ungleich 90 Grad relativ zu dem Bettschnitt bildet.

9. System nach Anspruch 8, wobei der Seitenschnitt einen Winkel von 30° bis 150° relativ zu dem Bettschnitt bildet.

10. System nach Anspruch 1, ferner umfassend einen gepulsten Laser, um den gepulsten Laserstrahl zu erzeugen, optional wobei eine Wiederholungsrate des gepulsten Lasers größer als 5 MHz ist und/oder wobei eine Pulsenergie des gepulsten Lasers kleiner als 100 nJ ist.

11. System nach Anspruch 1, wobei eine Frequenz des Hochfrequenzscanners größer als 1 kHz ist.

12. System nach Anspruch 1, wobei eine Länge der Abtastlinie in dem Bettschnitt, dem Ringschnitt und dem Seitenschnitt zwischen 400 und 1100 µm liegt.

13. System nach Anspruch 1, wobei die Steuerung konfiguriert ist, um den XY-Scanner und den Z-Scanner zu steuern, um die Abtastlinie derart abzutasten, dass eine während einer einzelnen Periode des Hochfrequenzscanners zurückgelegte Strecke weniger als 6 µm beträgt.

14. System nach Anspruch 1, wobei die Klappe eine elliptische Form aufweist.

15. System nach Anspruch 1, ferner umfassend, während Übergängen zwischen aufeinanderfolgenden Abtastdurchgängen des Bett- und Seitenschnitts, vorübergehende Austastimpulse des gepulsten Laserstrahls durch vorübergehendes Erhöhen einer Pulswiederholungsrate des gepulsten Laserstrahls, was die Pulsenergie des gepulsten Laserstrahls vorübergehend auf unter einen Photodisruptionsschwellenwert eines Hornhautgewebes reduziert und die Pulsdauer erhöht.

16. Computerpogrammpodukt, umfassend Anweisungen, die, wenn sie von einer Steuerung eines ophthalmologischen Chirurgielasersystems ausgeführt werden, bewirken, dass das ophthalmologische Chirurgielasersystem eine Hornhaut eines Patientenauges einschneidet, um eine Hornhautklappe zu bilden, durch:
Steuern eines Laserabgabesystems des ophthalmologischen Chirurgielasersystems, um einen gepulsten Laserstrahl an die Hornhaut abzugeben;
Steuern eines Hochfrequenzscanners des ophthalmologischen Chirurgielasersystems, um den gepulsten Laserstrahl hin und her abzutasten, um eine Laserabtastlinie zu bilden; und
Steuern eines Abtastlinienrotators, eines XY-Scanners und eines Z-Scanners des ophthalmologischen Chirurgielasersystems, um die Laserabtastlinie in der Hornhaut zu bewegen, um die Hornhautklappe zu bilden, einschließlich des Bildens eines Bettes der Klappe und des Bildens eines Seitenschnitts der Klappe und, vor dem Bilden des Bettschnitts, des Bildens eines Taschenschnitts,
wobei das Bett in einer horizontalen Ebene in einer ersten Tiefe von einer anterioren Hornhautoberfläche angeordnet ist, wobei das Bett eine Scharnierlinie definiert, wobei sich der Seitenschnitt von dem Bett nach oben zu der anterioren Hornhautoberfläche erstreckt, um eine Seite der Klappe zu bilden, wobei der Seitenschnitt eine gesamte Peripherie des Bettes mit Ausnahme der Scharnierlinie umgibt,
wobei das Bilden des Bettes der Klappe besteht aus:
Bilden eines Bettschnitts durch Abtasten der Laserabtastlinie in aufeinanderfolgenden überlappenden parallelen Rasterabtastdurchgängen, beginnend entlang der Scharnierlinie, und
Bilden eines einzelnen Ringschnitts entlang einer Peripherie des Bettes mit Ausnahme eines Bereichs des Scharnierschnitts durch Abtasten der Laserabtastlinie entlang eines Umfangs des Bettes, wobei der Ringschnitt alle Bereiche des Bettes abdeckt, die nicht durch den Bettschnitt abgedeckt sind,
und sich der Taschenschnitt von einer zweiten Tiefe posterior zu dem Bett bis zu der Tiefe des Bettes erstreckt, wobei der Bettschnitt mit dem Taschenschnitt verbunden ist;
wobei der Taschenschnitt einen Rampenbereich und einen Taschenbereich einschließt, die miteinander verbunden sind, wobei der Taschenbereich auf der zweiten Tiefe angeordnet ist, und wobei sich der Rampenbereich zwischen der ersten Tiefe und der zweiten Tiefe erstreckt und sowohl mit dem Bett als auch mit dem Taschenbereich verbunden ist.

## Revendications

1. Système laser chirurgical ophtalmique, comprenant :
un système de délivrance laser configuré pour délivrer un faisceau laser pulsé à une cornée d'un œil d'un patient ;
un dispositif de balayage haute fréquence (21) configuré pour balayer le faisceau laser pulsé vers l'arrière et vers l'avant à une fréquence prédéfinie pour former une ligne de balayage laser ;
un rotateur de ligne de balayage (23) configuré pour mettre en rotation une orientation de la ligne de balayage laser ;
un dispositif de balayage XY (28) et un dispositif de balayage Z (27) configurés pour déplacer la ligne de balayage laser dans des directions latérale et de profondeur ; et
une unité de commande (22) couplée fonctionnellement au, et programmée pour commander le rotateur de ligne de balayage, le dispositif de balayage XY et le dispositif de balayage Z pour balayer la ligne de balayage laser dans la cornée pour former un volet cornéen, y compris pour former un lit (202) du volet et pour former une coupe latérale (4) du volet, et pour former une coupe de poche (1) avant la formation de la coupe de lit,
dans lequel le lit est localisé dans un plan horizontal à une première profondeur par rapport à une surface cornéenne antérieure, le lit définissant une ligne de charnière (214), dans lequel la coupe latérale s'étend à partir du lit vers le haut jusqu'à la surface cornéenne antérieure pour former un côté du volet, la coupe latérale entourant une périphérie entière du lit à l'exception de la ligne de charnière,
dans lequel le lit du volet est constitué d'une coupe de lit (2) formée par balayage de la ligne de balayage laser en passes successives de balayage de trame parallèles se chevauchant, démarrant le long de la ligne de charnière, et une unique coupe annulaire (3) le long d'une périphérie du lit à l'exception d'une zone de la coupe de charnière formée par balayage de la ligne de balayage laser le long d'une circonférence du lit, dans lequel la coupe annulaire couvre toutes les zones du lit non couvertes par la coupe de lit, et
dans lequel la coupe de poche s'étend à partir d'une seconde profondeur postérieure au lit jusqu'à la profondeur du lit, dans lequel la coupe de lit est reliée à la coupe de poche ;
dans lequel la coupe de poche comporte une région de rampe (1B, 201B) et une région de poche (1A, 201A) reliées l'une à l'autre, dans lequel la région de poche est localisée à la seconde profondeur, et dans lequel la région de rampe s'étend entre la première profondeur et la seconde profondeur et est reliée à la fois au lit et à la région de poche.

2. Système selon la revendication 1, dans lequel toutes les coupes qui forment le volet cornéen sont mises en œuvre à une même énergie d'impulsion laser

3. Système selon la revendication 1, dans lequel la coupe latérale comporte une pluralité de couches de coupe latérale (4-1,4-2,4-3) formées dans une séquence, chaque couche de coupe latérale s'étendant au sein d'une plage de profondeur par rapport à la surface cornéenne antérieure, dans lequel toutes sauf une parmi la pluralité de couches de coupe latérale sont localisées entièrement au sein de la cornée sans atteindre la surface cornéenne antérieure, et dans lequel la pluralité de couches de coupe latérale sont alignées les unes avec les autres et se relient les unes aux autres pour former la coupe latérale.

4. Système selon la revendication 3, dans lequel chacune parmi la pluralité de couches de coupe latérale est formée en plaçant la ligne de balayage laser tangente à une circonférence de la coupe latérale, en déplaçant la ligne de balayage laser dans une direction verticale, en déplaçant simultanément la ligne de balayage laser autour de la circonférence, et en mettant en rotation simultanément la ligne de balayage pour la garder tangente à la circonférence.

5. Système selon la revendication 3, dans lequel des couches de coupe latérale adjacentes se chevauchent l'une l'autre dans une direction de profondeur.

6. Système selon la revendication 1, dans lequel une énergie d'impulsion laser de la coupe de lit et une énergie d'impulsion laser de la coupe latérale peuvent être réglées indépendamment.

7. Système selon la revendication 1, dans lequel chaque passe successive des passes de balayage de trame chevauche une passe de balayage précédente de 20 à 80 % d'une longueur des lignes de balayage.

8. Système selon la revendication 1, dans lequel la coupe latérale forme un angle différent de 90 degrés par rapport à la coupe de lit.

9. Système selon la revendication 8, dans lequel la coupe latérale forme un angle allant de 30° à 150° par rapport à la coupe de lit.

10. Système selon la revendication 1, comprenant en outre un laser pulsé pour générer le faisceau laser pulsé, facultativement dans lequel un taux de répétition du laser pulsé est supérieur à 5 MHz et/ou dans lequel une énergie d'impulsion du laser pulsé est inférieure à 100 nJ.

11. Système selon la revendication 1, dans lequel une fréquence du dispositif de balayage haute fréquence est supérieure à 1 kHz.

12. Système selon la revendication 1, dans lequel une longueur de la ligne de balayage dans la coupe de lit, la coupe annulaire et la coupe latérale est comprise entre 400 et 1100 µm.

13. Système selon la revendication 1, dans lequel l'unité de commande est configurée pour commander le dispositif de balayage XY et le dispositif de balayage Z pour balayer la ligne de balayage de telle sorte qu'une distance traversée pendant une période unique du dispositif de balayage haute fréquence est inférieure à 6 µm.

14. Système selon la revendication 1, dans lequel le volet a une forme elliptique.

15. Système selon la revendication 1, comprenant en outre, pendant des transitions entre des passes de balayage successives de la coupe de lit et latérale, l'occultation temporaire d'impulsions du faisceau laser pulsé en augmentant temporairement un taux de répétition d'impulsion du faisceau laser pulsé ce qui réduit temporairement l'énergie d'impulsion du faisceau laser pulsé en dessous d'un seuil de photodisruption d'un tissu cornéen et augmente la durée d'impulsion.

16. Produit programme informatique comprenant des instructions qui, lorsqu'elles sont exécutées par une unité de commande d'un système laser chirurgical ophtalmique, amènent le système laser chirurgical ophtalmique à inciser une cornée d'un œil d'un patient pour former un volet cornéen, par :
la commande d'un système de délivrance laser du système laser chirurgical ophtalmique pour délivrer un faisceau laser pulsé à la cornée ;
la commande d'un dispositif de balayage haute fréquence du système laser chirurgical ophtalmique pour balayer le faisceau laser pulsé vers l'arrière et vers l'avant pour former une ligne de balayage laser ; et
la commande d'un rotateur de ligne de balayage, d'un dispositif de balayage XY et d'un dispositif de balayage Z du système laser chirurgical ophtalmique pour déplacer la ligne de balayage laser dans la cornée pour former le volet cornéen, comportant la formation d'un lit du volet et la formation d'une coupe latérale du volet et avant la formation de la coupe de lit, la formation d'une coupe de poche,
dans lequel le lit est localisé dans un plan horizontal à une première profondeur par rapport à une surface cornéenne antérieure, le lit définissant une ligne de charnière, dans lequel la coupe latérale s'étend à partir du lit vers le haut jusqu'à la surface cornéenne antérieure pour former un côté du volet, la coupe latérale entourant une périphérie entière du lit à l'exception de la ligne de charnière,
dans lequel la formation du lit du volet est constituée de :
la formation d'une coupe de lit par balayage de la ligne de balayage laser en passes successives de balayage de trame parallèles se chevauchant, en démarrant le long de la ligne de charnière, et
la formation d'une unique coupe annulaire le long d'une périphérie du lit à l'exception d'une zone de la coupe de charnière par balayage de la ligne de balayage laser le long d'une circonférence du lit, dans lequel la coupe annulaire couvre toutes les zones du lit non couvertes par la coupe de lit,
et la coupe de poche s'étend à partir d'une seconde profondeur postérieure au lit jusqu'à la profondeur du lit, dans lequel la coupe de lit est reliée à la coupe de poche ;
dans lequel la coupe de poche comporte une région de rampe et une région de poche reliées l'une à l'autre, dans lequel la région de poche est localisée à la seconde profondeur, et dans lequel la région de rampe s'étend entre la première profondeur et la seconde profondeur et est reliée à la fois au lit et à la région de poche.
